# EUROPÄISCHE PATENTSCHRIFT

(11) **EP 2 349 130 B1**
(45) Veröffentlichungstag und Bekanntmachung des Hinweises auf die Patenterteilung: **19.03.2014**
(21) Anmeldenummer: 09768323.9
(22) Anmeldetag: 23.09.2009
(51) Int. Cl.: A61F 5/00

(54) **VORRICHTUNG ZUR STABILISIERUNG VON KÖRPERSEGMENTEN**
DEVICE FOR STABILIZING BODY SEGMENTS
DISPOSITIF POUR STABILISER DES SEGMENTS CORPORELS

(30) Priorität: 26.09.2008 DE 202008012828 U
(43) Veröffentlichungstag der Anmeldung: 03.08.2011
(60) Teilanmeldung: 13166013.6
(73) Patentinhaber: Gröhninger, Frank Friedrich, 66450 Oberbexbach (DE)
(72) Erfinder: Gröhninger, Frank Friedrich, 66450 Oberbexbach (DE)
(74) Vertreter: Wieske, Thilo
(86) Internationale Anmeldenummer: PCT/DE2009/075051
(87) Internationale Veröffentlichungsnummer: WO 2010/034307

(56) Entgegenhaltungen:
- US-A- 5 628 721
- US-A- 5 695 452
- US-A- 5 792 084
- US-A1- 2007 167 895

## Beschreibung

Die Erfindung betrifft eine Vorrichtung zum Stabilisieren von Körpersegmenten.

Aus der US 6,779,282 B2 ist ein Sportstrumpf mit einer sich von den Zehen bis zur Ferse erstreckenden Einlage bekannt, die eine steigbügelartige Vorrichtung in Form zweier miteinander verbundener Streifen aus geeignetem Material umfaßt, welche miteinander verbunden sind um den Knöchel zu stabilisieren und die ein senkrecht zu den Streifen angeordnetes, horizontal umlaufendes Band aufweist, das zu einer Bewegungsbescbränkung des Talocalcaneonavikulargelenks und Stabilisieren des Knöchels dient. Hierbei ist vorgesehen, daß die Einlage mit dem Spottstrumpf unter anderem durch Sprühbinden mit Kunststoff verbunden ist. Es kann auch eine Kunststoffschicht auf den beiden Streifen vorgesehen sein. Bei Verwendung dieser Vorrichtung kann das "Tapen", d.h. das Stabilisieren mit Tape-Band entfallen.

Aus der US 5,695,452 A ist eine orthopädische Shützvorrichtung bekannt, der aus einem in Teilbereichen drurkgeformten Schaummaterial besteht, wobei die Teilbereiche eine andere Dicke und Dichte aufweisen als die übrigen Bereiche.

Es sind weiterhin Gipsverbände zum Festlegen von Körpersegmenten bekannt, welche weitgehend im Bereich der Medizin, insbesondere bei Knochenbrüchen, zur Anwendung kommen. Diese müssen jedoch individuell angelegt werden, was aufwendig ist.

Es sind schließlich auch Kunststoffschalverbände bekamt, welche ebenfalls im Bereich der Medizin verwendet werden und in Anbetracht ihres geringeren Gewichtes, den Gipsverbänden zunehmend bevorzugt werden.

Aufgabe der Erfindung ist es, insbesondere für die Anwendung im Bereich des Sports und der Rehabilitationsmedizin eine neuartige Vorrichtung zum/ Stabilisieren von Körpersegmenten zu schaffen, die als vorgefertigte Konfektionsware oder auch als halbfertige Maßkonfektion vertrieben werden kann.

Diese Aufgabe wird erfindungsgemäß dadurch gelöst, daß zur Ausbildung eines anatomisch an den jeweiligen Körpersegmenten anliegenden orthopädischen Hilfsmittels auf eine textile Trägerschicht, welche die Form des zu stabilisierenden Körpersegmentes aufweist, eine diese in ihrer Form stabilisierende Kunststoffschicht aufgetragen ist, wobei ein Einlegestück von der Kunststoffschicht umgeben ist, sodaß mit der Vorrichtung ein oder mehrere an das jeweilige Körpersegment angrenzende weitere Körpersegmente und das bzw. die dazwischen liegende(n) Gelenk(e) mit abdeckbar sind, und wobei Mittel zur Beschränkung des Bewegungswinkels des bzw. der Gelenke vorgesehen sind.

Es wird im Rahmen der Erfindung ein funktionelles anatomisch kongruent an den jeweiligen Körpersegmenten anliegendes orthopädisches Hilfsmittel geschaffen. Während bei derzeit am Markt erhältlichen Bandagen eine natürliche konkave Körperüberspannung (beispielsweise im Bereich der Lendlordose) hervorgerufen wird, so daß dort zur Druckausübung eine Pelotte eingesetzt werden muß, folgt die erfindungsgemäße Vorrichtung in allen Bereichen, auch bei konkaven Körperüberspannungen der Körperkontur, wodurch eine bessere Paßform erreicht wird. Vielmehr kann individuell eingestellt werden, wo Druck auf den Körper ausgeübt werden soll und wo nicht. Auf diese Weise wird eine Vorrichtung zum Stabilisieren von Körpersegmenten geschaffen, die als Orthesen-Bandage oder als Handagen-Orthese bezeichnet werden kann und bei geringem Gewicht einen hohen Tragekomfort aufweist, da die textile Trägerschicht der Haut des Benutzers zugewandt ist. Mit dieser Vorrichtung können Bewegungen der verschiedenen Gelenke ganz oder teilweise eingeschränkt, stabilisiert oder unterstützt werden, was auch zur Erhöhung der natürlichen Biodynamik genutzt werden kann.

Die stabilisierende Kunststoffschicht kann netzartig oder flärbig, flexibel oder starr, elastisch, hart oder weich, zwei- oder dreidimensional sein, wobei es auch möglich ist, mehrere dieser Eigenschaften in einer Vorrichtung miteinander zu kombinieren, beispielsweise durch die gezielte Verwendung unterschiedlicher Kunststoffschichten für verschiedene Bereiche der Vorrichtung, beispielsweise ein bestimmtes Gelenk unterstützend und ein anderes nur teilunterstützend. Ebenso kann die Kunststoffschicht ein- oder mehrlagig ausgebildet sein. Die Kunststoffschicht ist aufgrund ihrer Verbindung mit der textilen Trägerschicht unverrückbar positioniert. Sie kann sowohl außen als auch innen am textilen Trägergewebe aufgespritzt sein und mit einer Rutschhilfe abgedeckt werden. Es ist selbstverständlich möglich, zusätzlich weiche Kunststoffapplikationen, beispielsweise aus Silikon, als Pelotte (Druckpolster) auf der Vorrichtung anzuordnen, um gezielt Druck auf bestimmte Körperregionen auszuüben. Das biegbare Metallelement ermöglicht es, die noch formbare Vorrichtung nach dem eigentlichen Herstellungsvorgang individuell formgebend einem Körperteil anzupassen.

Es versteht sich, daß nicht nur das angrenzende Gelenk mit von der Vorrichtung abgedeckt wird, sondern mindestens teilweise auch das an dieses Gelenk angrenzende weitere Körpersegment. Bei einer Vorrichtung, die zur Stabilisierung des Oberkörpers und eines Arms vorgesehen ist, wäre somit der Oberkörper zumindest teilweise, das Schultergelenk und ein Teil des Oberarmknochens abgedeckt.

Die Körpersegmente können die oberen oder unteren Gliedmaßen oder der Rumpf sein.

Erfindungsgemäß ist vorgesehen, daß das Einlegestück aus Metall besteht.

In diesem Zusammenhang ist es vorteilhaft, daß das Einlegestück ein biegsames Metallelement ist.

Ein solches Einlegestück kann beispielsweise aus einem biegsamen Metallelement bestehen, das es ermöglicht, die dann noch formbare Vorrichtung nach dem eigentlichen Herstellungsvorgang individuell formgebend einem Körperteil anzupassen.

Es ist ebenfalls möglich, daß das Einlegestück aus Kunststoff besteht.

Es liegt im Rahmen der Erfindung, daß die textile Trägerschicht ein textiles Gewirk oder ein Gewebe ist.

Grundsätzlich kommen beide Alternativen in Frage. Die textile Trägerschicht kann auch elastisch ausgebildet sein, beispielsweise als elastisches Textilgewirk oder -gewerbe oder als Textilgummigewirk oder Kombination derselben. Grundsätzlich kommen auch textilähnliche Strukturen, wie beispielsweise Neopren in Frage.

Eine Weiterbildung der Erfindung besteht darin, daß die Vorrichtung mehrlagig aufgebaut ist.

Die Vorrichtung kann starr, elastisch oder flexibel ausgebildet sein.

Es ist möglich, die Vorrichtung nicht nur einlagig, sondern auch mehrlagig auszubilden.

Ebenfalls im Rahmen der Erfindung liegt es, daß mindestens ein Stabilisierungsband zum Erhöhen der Haftung vorgesehen ist.

Ein solches Stabilisierungsband kann sowohl statisch fest als auch elastisch fixierend oder diese beiden Eigenschaften kombinierend ausgebildet sein.

Ebenfalls ist es erfindungsgemäß vorgesehen, daß an der dem Körper des Benutzers zugewandten Innenseite eine über ein Ventil mit Luft befüllbare Druckkammer vorgesehen ist.

Durch das Aufpumpen einer solchen Druckkammer kann das Anliegen der Vorrichtung am Körper noch verbessert werden, durch Luftablassen können Schwellungen kompensiert werden oder aber es kann Druck auf die stabilisierten Körpersegment ausgeübt werden.

Nachfolgend wird ein Ausführungsbeispiel der Erfindung anhand von Zeichnungen beschrieben.

Es zeigt
- Fig. 1: eine schematische Darstellung des Aufbaus der Vorrichtung,
- Fig. 2: eine schematische Darstellung einer erfindungsgemäßen Vorrichtung zur Stabilisierung von Ober- und Unterschenkel und das damit verbundene Kniegelenk in M-L, A-P und Rotationsbewegung.

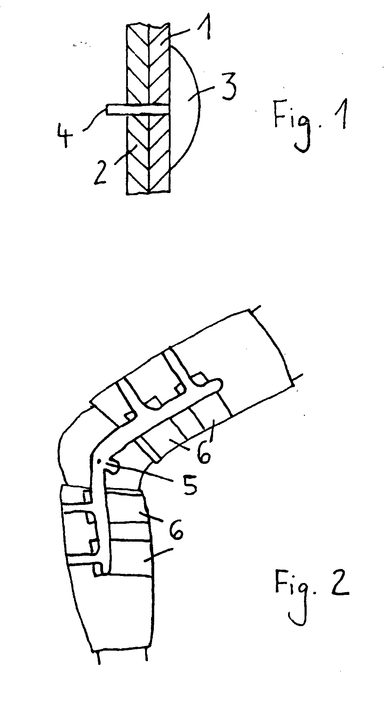

Wie aus Fig. 1 ersichtlich, weist die erfindungsgemäße Vorrichtung eine der Form des bzw. der betroffenen Körpersegmente angepaßte textile Trägerschicht 1 auf, bei der es sich um ein textiles Gewirk oder ein textiles Gewebe handeln kann. Auf diese textile Trägerschicht 1 ist eine die textile Trägerschicht 1 in ihrer Form stabilisierende Kunststoffschicht 2 aufgetragen ist. Hierbei kann es vorgesehen sein, daß in bestimmten hoch beanspruchten Bereichen oder über die gesamte Vorrichtung hinweg dieser Schichtaufbau sandwichartig wiederholt wird, bis die gewünschte mechanische Belastbarkeit erreicht ist.

Weiterhin kann die Vorrichtung eine mit Luft befüllbare Druckkammer 3 aufweisen, die es ermöglicht, den Sitz der Vorrichtung am betreffenden Körpersegment zu verbessern oder aber während des Tragens durch Druckablassen den Sitz der Vorrichtung an dem Körpersegment etwas zu lockern. Diese Druckkammer 3 weist ein Ventil 4 auf, über das die Druckkammer 3 befüllt bzw. entleert werden kann.

Die dargestellte Vorrichtung weist ebenfalls Mittel 5 zur Beschränkung des Bewegungswinkels des Kniegelenks auf, die als mit beiden Teilen der Vorrichtung verbundenes Gelenk 5 zwischen dem den Oberschenkel umfassenden und dem den Unterschenkel umfassenden Teil der Vorrichtung ausgebildet sind, welches einen einstellbaren maximalen Beugewinkel aufweist. Die Fixierbänder 6 können in horizontaler Richtung bzw. in jeder anderen Lagenform angebracht sein.

## Patentansprüche

1. Vorrichtung zum Stabilisieren von Körpersegmenten, wobei zur Ausbildung eines anatomisch an den jeweiligen Körpersegmenten anliegenden orthopädischen Hilfsmittels auf eine textile Trägerschicht (1), welche die Form des zu stabilisierenden Körpersegmentes aufweist, eine diese in ihrer Form stabilisierende Kunststoffschicht (2) aufgetragen ist, wobei ein Einlegestück von der Kunststoffschicht (2) umgeben ist, sodaß mit der Vorrichtung ein oder mehrere an das jeweilige Körpersegment angrenzende weitere Körpersegmente und das bzw. die dazwischen liegende(n) Gelenk(e) mit abdeckbar sind, und wobei Mittel (5) zur Beschränkung des Bewegungswinkels des bzw. der Gelenke vorgesehen sind.

2. Vorrichtung gemäß Anspruch 1, wobei das Einlegestück aus Metall besteht.

3. Vorrichtung gemäß Anspruch 2, wobei das Einlegestück ein biegsames Metallelement ist.

4. Vorrichtung gemäß Anspruch 1, wobei das Einlegestück aus Kunststoff besteht.

5. Vorrichtung gemäß Anspruch 1, wobei die textile Trägerschicht (1) ein textiles Gewirk oder ein Gewebe ist.

6. Vorrichtung gemäß Anspruch 1, wobei die Vorrichtung mehrlagig aufgebaut ist.

7. Vorrichtung gemäß Anspruch 1, wobei mindestens ein Stabilisierungsband zum Erhöhen der Haftung vorgesehen ist.

8. Vorrichtung gemäß Anspruch 1, wobei an der dem Körper des Benutzers zugewandten Innenseite eine über ein Ventil mit Luft befüllbare Druckkammer (3) vorgesehen ist.

## Claims

1. Device for stabilizing body segments, wherein, for the purpose of engineering an orthotic device that makes anatomically congruent contact with the respective body segments, a textile substrate (1) having the shape of the body segment to be stabilized has an anatomically congruent layer of plastic (2) applied thereon that stabilizes the shape of the textile substrate, wherein an insert is surrounded by said layer of plastic (2) so that one or more additional body segments adjacent to the body segment in question, as well as the joint(s) therebetween, can be covered by the device, and wherein means (5) to restrict the angle of movement of the joint(s) are provided.

2. Device according to claim 1, wherein the insert consists of metal.

3. Device according to claim 2, wherein the insert is a flexible metal element.

4. Device according to claim 1, wherein the insert consists of plastic.

5. Device according to claim 1, wherein the textile substrate (1) is a knitted textile fabric or a woven textile fabric.

6. Device according to claim 1, wherein the device is made up of a plurality of layers.

7. Device according to claim 1, wherein at least one stabilizing strap is provided to enhance adhesion.

8. Device according to claim 1, wherein, on the inside of the device, facing the wearer's body, a pressure chamber (3) is provided which can be inflated with air via a valve.

## Revendications

1. Dispositif pour stabiliser des segments corporels, dans lequel, pour réaliser un dispositif orthopédique qui épouse de façon anatomique les segments corporels considérés, on applique sur une couche support textile (1) qui présente la forme du segment corporel à stabiliser une couche synthétique (2) stabilisant la forme de la couche support, un insert étant entouré par la couche synthétique (2) de sorte qu'un ou plusieurs autres segments corporels adjacents au segment corporel considéré et la ou les articulations situées entre ceux-ci peuvent être également couverts par le dispositif, et des moyens (5) étant prévus pour limiter l'angle de mouvement de la ou les articulations.

2. Dispositif selon la revendication 1, dans lequel l'insert est en métal.

3. Dispositif selon la revendication 2, dans lequel l'insert est un élément métallique flexible.

4. Dispositif selon la revendication 1, dans lequel l'insert est en matière plastique.

5. Dispositif selon la revendication 1, dans lequel la couche support textile (1) est un tricot textile ou un tissu.

6. Dispositif selon la revendication 1, dans lequel le dispositif est constitué de plusieurs couches.

7. Dispositif selon la revendication 1, dans lequel au moins une bande de stabilisation est prévue pour augmenter l'adhésion.

8. Dispositif selon la revendication 1, dans lequel un compartiment de pression (3) pouvant être rempli d'air par le biais d'une valve est prévu sur la face interne dirigée vers le corps de l'utilisateur.
